# EUROPÄISCHE PATENTSCHRIFT

(11) **EP 2 110 156 B1**
(45) Veröffentlichungstag und Bekanntmachung des Hinweises auf die Patenterteilung: **02.11.2016**
(21) Anmeldenummer: 09155360.2
(22) Anmeldetag: 17.03.2009
(51) Int. Cl.: A61N 1/08

(54) **Feldentkopplungs-Element zur Verwendung mit einer implantierbaren Leitung und implantierbares medizinisches Gerät**
Field decoupling element for use with an implantable lead and implantable medical device
Elément de découplage de champs à utiliser avec une sonde implantable et appareil médical implantable

(30) Priorität: 14.04.2008 DE 102008018992
(43) Veröffentlichungstag der Anmeldung: 21.10.2009
(73) Patentinhaber: Biotronik CRM Patent AG, 6341 Baar (CH)
(72) Erfinder: Weiss, Ingo, 12435, Berlin (DE); Knorr, Stefan, 10119, Berlin (DE); Maxfield, Michelle, 10967, Berlin (DE); Friedrich, Michael, 14532, Kleinmachnow (DE)
(74) Vertreter: Galander, Marcus

(56) Entgegenhaltungen:
- WO-A2-03/037424
- US-A1- 2005 222 657
- US-A1- 2006 247 747
- US-A1- 2006 252 314

## Beschreibung

Die Erfindung betrifft ein Feldentkopplungs-Element zur Verwendung mit einer implantierbaren Leitung mit einem langgestreckten Leitungskörper und einem sich in Längsrichtung des Leitungskörpers erstreckenden Funktionsleiter, der zur Realisierung einer medizinischen Funktion der Leitung wirkt. Sie betrifft weiter eine implantierbare Leitung der genannten Art sowie ein implantierbares medizinisches Gerät zum Anschluss einer derartigen implantierbaren Leitung. Medizinische Implantate, wie Schrittmacher und Defibrillatoren, besitzen häufig eine elektrische Verbindung zum Körperinneren des Patienten. Eine solche Verbindung dient zur Messung von elektrischen Signalen und/oder zur Stimulation von Körperzellen. Diese Verbindung ist oft als längliche Elektrode ausgeführt. Gegenwärtig werden elektrische Signale zwischen dem Implantat und den Elektrodenkontakten wie, aber nicht beschränkt auf Spitze, Ringe, HV-Schockwendeln und Sensoren mit elektrisch gut leitenden Materialen übertragen.

Wird ein System aus Implantat und Elektrode starken Störfeldern (EMI, MRI) ausgesetzt, so kann es zu unerwünschtem Fehlverhalten kommen; speziell einer Erwärmung von Teilen des Systems oder elektrische Fehlfunktionen (z. B. Resets). Die Erwärmung kann zu Schädigungen von Körpergewebe oder von Organen führen, wenn die erwärmten Teile direkten Kontakt zum Gewebe haben. Dies ist insbesondere bei der Elektrodenspitze der Fall.

Die Ursache für das unerwünschte Fehlverhalten ist die Wechselwirkung des Feldes mit der länglichen Leitungsstruktur der Elektrode: Die Elektrode wirkt als eine Antenne und empfängt Energie aus den umgebenden Feldern. Diese Energie auf den therapeutisch genutzten Leitungen kann die Antenne distal über die Elektrodenkontakte (Spitze, Ring ..) an das Gewebe oder proximal an das Implantat abgeben.

Die gleichen Probleme treten auch bei anderen länglichen leitfähigen Strukturen auf, deren proximales Ende nicht zwingend mit einem Implantat verbunden ist (z. B. Kathetern, temporären Elektroden).

Die Dokumente WO03/037424 und US2005/0222657 offenbaren Feldentkopplungselemente, die permanent mit einem Leiter verbunden sind.

Der Erfindung liegt die Aufgabe zugrunde, eine verbesserte implantierbare Leitung der genannten Art bereit zu stellen, die in starken äußeren Feldern verbesserte Eigenschaften aufweist.

Die Aufgabe wird durch die Bereitstellung eines Feldentkopplungs-Elementes mit den Merkmalen des Anspruchs 1 gelöst.

Ein wesentlicher Gedanke der Erfindung besteht darin, ein mit der implantierbaren Leitung zu verbindendes oder in diese integriertes Element bereit zu stellen, welches die Wechselwirkung der Leitungsstruktur, d. h. des oder der Funktionsleiter(s), mit externen Störfeldern in einem Sinne gezielt verändert, dass sich als Resultat die Antennenwirkung der Leitungsstruktur verschlechtert. Das vorgeschlagene Element wird daher als Feldentkopplungs-Element bezeichnet, wobei dieser Begriff nicht das Erreichen einer vollständigen Entkopplung der Leitungsstruktur von äußeren Feldern, sondern lediglich eine graduelle Entkopplung benennen soll.

Im Gebrauchszustand hat das Feldentkopplungs-Element Kontakt zu mindestens einem Funktionsleiter der implantierbaren Leitung und kann zusätzlich Kontakt zu weiteren leitfähigen Elementen oder Bereichen haben, etwa zu Körperflüssigkeit, der Haut, weiteren implantierten Leitern - darunter dem Gehäuse eines implantierbaren medizinischen Gerätes -, zu einem Bezugspotential (z. B. Erde), etc.. Unter einem Kontakt ist hier im allgemeinen Sinne eine elektrische Verbindung mit geringer Impedanz zu verstehen, die bei hoch frequenten Signalen durch eine kapazitive Kopplung gegeben sein kann.

Vorgeschlagen wird insbesondere ein Feldentkopplungs-Element mit mindestens zwei Kontakten zur Kontaktierung des Funktionsleiters der Leitung sowie eines weiteren leitenden Elementes im Körper eines Patienten und einem beide Kontakte verbindenden Schaltnetzwerk. Dieses Netzwerk besteht aus z. B.
- diskreten Bauelemente: Widerstände, Kondensatoren, Induktivitäten, die ein elektrisches Netzwerk bilden,
- leitenden oder dielektrischen Materialien (Kunststoffe, Metalle, Keramiken),
- Sensoren (Thermoelement, Thermistor, Feldstärkesensor,...),
- Halbleiterstrukturen,
- Nichtlinearen Bauelementen / Strukturen,
- Bauelementen, die einen Riesenmagnetwiderstand (GMR), anisotropen Magnetwiderstand (AMR), kolossalen Magnetwiderstand (CMR) oder magnetischen Tunnelwiderstand (TMR) besitzen,
- oder durch Kombinationen oben genannter Verbindungen.

In einer weiteren Ausführung der Erfindung ist das Feldentkopplungs-Element mit einem elastischen leitfähigen Abschnitt oder Teil versehen, welcher/welches im Gebrauchszustand den Funktionsleiter einer angeschlossenen Leitung auch bei mechanischer Krafteinwirkung in Dauerkontakt mit einem weiteren leitenden Element im Körper eines Patienten hält. Weiterhin kann vorgesehen sein, dass das Feldentkopplungs-Element einen Abschnitt oder ein Teil aus elektrischem leitfähigem Kunststoff aufweist, welcher/welches insbesondere den ersten mit dem zweiten Kontakt verbindet. Alternativ hierzu oder auch in Kombination hiermit kann vorgesehen sein, dass das Feldentkopplungs-Element einen metallischen Abschnitt oder ein metallisches Teil aufweist, welcher/ welches den ersten mit dem zweiten Kontakt verbindet und in den/das insbesondere ein verstellbares oder elastisches Kontaktierungselement eingefügt ist.

Durch die Materialwahl lässt sich eine gezielte Einstellung der mechanischen und elektrischen Eigenschaften des Feldentkopplungs-Elementes erreichen, mit der unterschiedlichste Leitungsstrukturen hinsichtlich ihrer Performance in starken externen Feldern wesentlich verbessert und vielfältigste Anwendungen abgedeckt werden können. In diesem Sinne kann weiterhin vorgesehen sein, dass ein aus hoch leitfähigem Material bestehender Oberflächenabschnitt mit einer Isolierschicht bedeckt ist, welche insbesondere aus einem solchen Material besteht und/oder so bemessen ist, dass sie eine vorbestimmte niedrige Impedanz aufweist.

In einer weiteren Ausführung der Erfindung ist die Nutzung einer leitfähigen Flüssigkeit zur Vermittlung des erwünschten Kontakts zwischen einem Funktionsleiter und weiteren implantierten leitenden Teilen vorgesehen. Ein entsprechendes Feldentkopplungs-Element weist insbesondere einen mindestens den ersten mit dem zweiten Kontakt verbindenden Hohlraum zur Aufnahme einer leitfähigen Flüssigkeit auf, welcher insbesondere derart ausgebildet ist, dass im Gebrauchszustand Körperflüssigkeit eines Patienten eindringen kann und eine elektrische Verbindung zwischen den Kontakten herstellt. In diesem Sinne wird das Feldentkopplungs-Element also erst im Gebrauchszustand durch Eindringen von Körperflüssigkeit strukturell und funktionell komplettiert.

In einer weiteren Ausführung der Erfindung ist das Feldentkopplungs-Element ausgebildet zur Verbindung mit mehreren Leitungen und/oder mehreren implantierbaren medizinischen Geräten. Es kann in dieser Ausführung eine besonders einfache und nützliche Ergänzung z. B. einer Schrittmacheranordnung mit Leitungen zur atrialen und ventrikulären Stimulation oder eines kombinierten Herzschrittmachers/Defibrillators oder anderer Kombinationsgeräte darstellen.

Das vorgeschlagene Feldentkopplungs-Element, welches unter Bezugnahme auf die neuerdings eingebürgerte Bezeichnung "Elektrode" für implantierbare Elektrodenleitungen und unter Hinweis auf seinen üblichen Anbringungsort auch als "proximale Elektrodenterminierung" bezeichnet werden kann, kann in verschiedenen Ausführungen eine Vielzahl sinnvoller Aspekte haben. Es kann sich insbesondere um eine Elektrodenterminierung handeln,
- die implantierbar ist;
- deren Bestandteile ganz oder teilweise biokompatibel sind;
- wobei die Terminierung das Körpergewebe mit mindestens einem der elektrischen Anschlüsse eines Elektrodensteckers (z. B. IS1, IS4, DF1) über ein elektrisches Netzwerk verbindet;
- wobei die Terminierung Kontakt zu allen Anschlüssen einer Elektrode besitzt und zusätzlich einen Kontakt zum umgebenden Körpergewebe besitzt;
- wobei die elektrische Verbindung zum Körpergewebe nicht galvanisch, sondern kapazitiv erfolgt;
- wobei die Terminierung auf mindestens einen der Standard-Elektrodenanschlüsse (IS1, IS4, DF1) passt;
- wobei der Kontakt zwischen der Terminierung und dem Elektrodenstecker durch eine nachgiebige Teilstruktur (z. B. Lamellen oder Federn) der Terminierung und/oder durch eine Schraubverbindung gewährleistet wird;
- wobei die Terminierung mehrere Elektrodenstecker aufnehmen kann, die zu verschiedenen Elektroden gehören können (ICD Elektrode oder mehrere bipolare Elektroden);
- die einen hoch leitfähigen Kontakt zum umgebenden Gewebe besitzt, z. B. durch ein Metall, einen leitfähigen Kunststoff, einen Kunststoff mit leitfähigen Partikeln oberhalb der Perkulationsschwelle oder andere Substanzen hergestellt werden kann;
- die aus einem Kunststoff mit geringer Impedanz besteht, der mehrere Eingänge der Terminierung verbindet, z. B. Silikon oder Polyurethan sein, ggf. gefüllt mit verschiedenen leitfähigen Materialien z. B. Kohlenstoff, Ruß, Fulleren, Nanotubes, Metallstäuben, Bariumtitanat, Aluminium, Wolfram, Gold, Magnesium, Tantal ... );
- wobei die Impedanz des Kunststoffes frequenzabhängig ist;
- die mindestens teilweise aus einem weichen, elastischen Kunststoff besteht;
- die ein metallisches Gehäuse besitzt;
- deren hoch leitfähige Oberfläche ganz oder teilweise isoliert ist, wobei die Isolierung so gestaltet sein kann, dass die Impedanz sehr gering ist (z. B. durch eine besonders dünne Isolierung);
- die keine galvanische Verbindung zum Körpergewebe besitzt, d. h. wobei die Kopplung zum Körpergewebe nur kapazitiv erfolgt;
- wobei einige der elektrischen Eingänge (Gewebe, Leitung(en) der 1. Elektrode, Leitung(en) der 2. Elektrode, ..) durch eine geringe Impedanz miteinander verbunden sein können, z. B. als niederohmige oder kapazitive Struktur;
- wobei die elektrische Verbindung durch eine leitfähige Flüssigkeit gewährleistet wird;
- wobei die elektrische Verbindung durch Körperflüssigkeit gewährleistet wird;
- wobei die Terminierung Öffnungen besitzt, die einen direkten Kontakt zwischen den Elektrodenanschlüssen und dem Körpergewebe erlauben;
- deren Elektrodenanschluss (IS1, IS4, DF1) neben den üblichen Kontakten einen weiteren Anschluss für einen im Innenlumen der Elektrode platzierten Leiter (MRIwire) besitzt, wobei der zuverlässige elektrische Kontakt zu diesem Leiter durch eine federnde Struktur gewährleistet werden kann;
- wobei die Terminierung ein aktives Implantat (Schrittmacher, ICD, Stimulator ...) ist;
- wobei die äußere Form der Terminierung am proximalen Ende so geformt ist, dass sie das umgebende Gewebe nicht verletzen kann;
- wobei die Terminierung auch Kontakt zu einer elektrischen Leitungsstruktur hat, die in das Innenlumen der Elektrode passt;
- wobei die Terminierung fest mit einer elektrischen Leitungsstruktur verbunden ist, die in das Innenlumen der Elektrode passt;
- wobei die Terminierung nicht nur Anschlüsse für Elektroden besitzt, sondern noch einen weiteren Anschluss zur Kontaktierung einer anderen Terminierung (z. B. eines anderen aktiven Implantates);
- wobei die Terminierung nicht nur Anschlüsse (Buchsen) für Elektroden besitzt, sondern noch mindestens einen weiteren Elektrodenstecker und/oder andere Kontaktierungsmöglichkeit für ein anderes Implantat (z. B. Gehäusekontakt);
- die fest mit einer anderen Terminierung (z. B. ICD) verbunden werden kann, so dass die Lage zwischen der Elektrode an der Terminierung und z. B. einem ICD festgelegt und der Einfluss der Elektrode auf die Funktion des ICD zeitlich konstant gehalten werden kann;
- die Anschlüsse für mehrere Elektroden besitzt, von denen mindestens eine nicht zu Therapiezwecken verwendet wird (z. B. ein Schrittmacher, an den eine nicht mehr genutzte Elektrode angeschlossen werden kann);
- die auf gekürzte Elektroden gesteckt werden kann;
- die nicht auf das Ende oder das gekürzte Ende einer Elektrode gesteckt wird, sondern entlang der Elektrode montiert wird;
- die eine längliche Form besitzt, so dass ein guter Kontakt (galvanisch, kapazitiv) zum umgebenden Körpergewebe hergestellt werden kann;
- die eine längliche Form besitzt, und nach der Montage hauptsächlich um die Elektrode herum angeordnet ist (wie ein Schlauch, der über die Elektrode geschoben wird);

Die Erfindung ermöglicht die Realisierung von implantierbaren Leitungen und insgesamt implantierbaren medizinischen und speziell medizinisch elektronischen Systemen der oben erwähnten Art mit verbesserten Störfeldeigenschaften, deren Elektroden bzw. Gewebekontakten sich insbesondere in starken externen Feldern (etwa bei einer MR-Untersuchung) nur schwach erwärmen. In Ausführung der Erfindung wird insbesondere die nachträgliche Umrüstung bereits implantierbarer Leitungen durch einen relativ einfachen Eingriff möglich, um diesen die genannten verbesserten Eigenschaften zu verleihen.

Vorteile und Zweckmäßigkeiten der Erfindung ergeben sich im Übrigen aus der nachfolgenden Beschreibung von Ausführungsbeispielen anhand der Figuren.

Von diesen zeigen:
- Fig. 1A bis 1C: Prinzipskizzen verschiedener grundsätzlicher Ausführungsmöglichkeiten der Erfindung,
- Fig. 2: eine schematische Darstellung des proximalen Endes einer implantierbaren Leitung, eingesetzt in eine Steckbuchse eines implantierbaren medizinischen Gerätes und versehen mit einem Feldentkopplungs-Element,
- Fig. 3A bis 3C: schematische Längsschnittdarstellungen der proximalen Enden von implantierbaren Leitungen mit verschiedenen Ausführungen eines Feldentkopplungs-Elementes,
- Fig. 4: eine schematische Längsschnittdarstellung einer weiteren Ausführung der Erfindung,
- Fig. 5A bis 5C: Prinzipskizzen weiterer Ausführungsmöglichkeiten der Erfindung,
- Fig. 6: eine schematische Längsschnittdarstellung einer verzweigten implantierbaren Leitung zur Realisierung der in Fig. 5A skizzenartigen dargestellten Ausführungsform,
- Fig. 7A und 7B: Abwandlungen der in Fig. 2 dargestellten Ausführung zum Anschluss mehrerer Elektrodenleitungen oder einer mehrpoligen Elektrodenleitung,
- Fig. 8A bis 8D: Prinzipdarstellungen weiterer Ausführungsmöglichkeiten der Erfindung,
- Fig. 9A bis 9C: eine skizzenartige perspektivische Darstellung bzw. schematische Längsschnittdarstellungen einer weiteren grundsätzlichen Ausführungsmöglichkeit der Erfindung,
- Fig. 10: eine Prinzipskizze des Innenaufbaus eines Herzschrittmacher-Headers mit einem Aufbau gemäß einer Ausführung der Erfindung und
- Fig. 11A und 11B: Prinzipskizzen einer weiteren Ausführungsform der Erfindung unter zwei verschiedenen Aspekten.

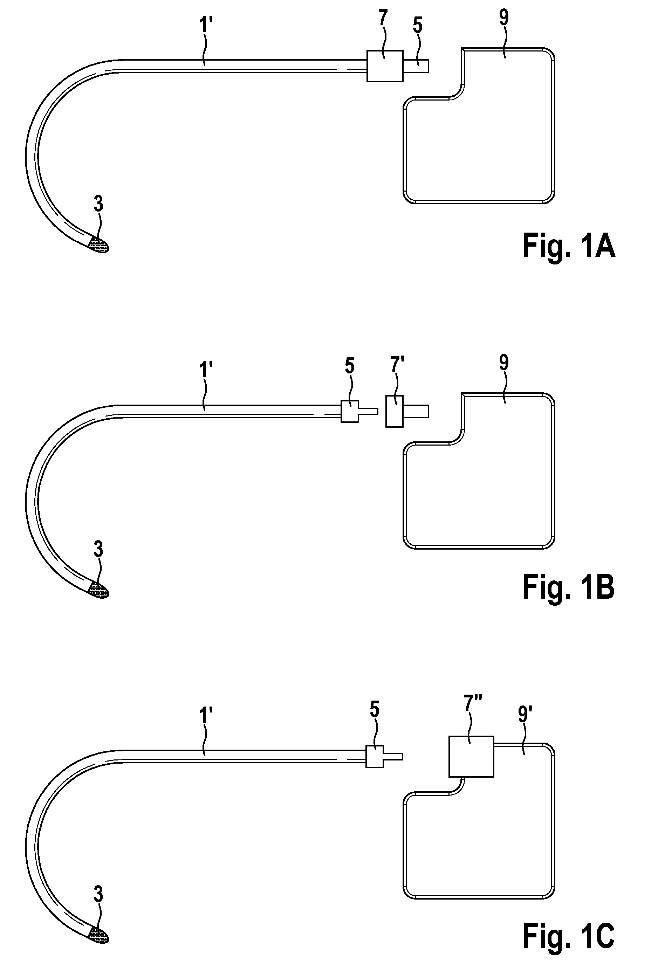

Fig. 1A zeigt schematisch eine implantierbare Stimulationselektrodenleitung 1' mit einer Spitzenelektrode 3 und einem proximalen Elektrodenanschlusskontakt 5, die ein am proximalen Ende integriertes Feldentkopplungs-Element 7 enthält, zum Anschluss an einen herkömmlichen Herzschrittmacher 9. Fig. 1B zeigt eine weitere Ausführung der Erfindung, bei der ein separates Feldentkopplungs-Element 7' zum nachträglichen Anfügen an eine herkömmliche Stimulationselektrodenleitung 1' mit Spitzenelektrode 3 und Elektrodenanschlusskontakt vorgesehen ist, wobei das Feldentkopplungs-Element bei dem Anschluss der Leitung an den Herzschrittmacher 9 zwischen den Elektrodenanschlusskontakt 5 und einen (nicht gesondert bezeichneten) Header des Herzschrittmachers eingefügt wird. Fig. 1C schließlich zeigt eine dritte Ausführungsmöglichkeit, bei der die herkömmliche Stimulationselektrodenleitung 1' mit einem modifizierten Herzschrittmacher 9' verbunden wird, in dessen Anschlussbereich ein entsprechend gestaltetes Feldentkopplungs-Element 7" eingefügt ist.

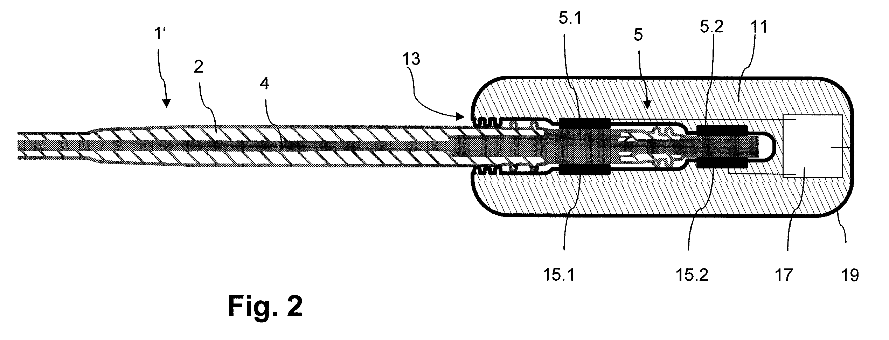

Fig. 2 zeigt in einer vereinfachten Darstellung (die z. B. das Vorhandensein mehrerer Elektrodenzuleitungen nicht erkennen lässt) den proximalen Endabschnitt einer herkömmlichen Stimulationselektrodenleitung 1' mit einem Leitungskörper 2, einem Funktionsleiter 4 und einem Steckerabschnitt 5 mit einem ersten und zweiten Elektrodenanschlusskontakt 5.1 und 5.2, eingeführt in eine Anschlussbuchse 11 eines medizinelektronischen Gerätes mit einer Steckeraufnahme 13 und einem ersten und zweiten Steckbuchsenkontakt 15.1 und 15.2. Die Steckbuchsenkontakte 15.1 und 15.2 sind über ein hier symbolisch dargestelltes Feldentkopplungs-Element 17 miteinander und mit einem leitfähigen Gehäuse 19 verbunden. Das Feldentkopplungs-Element enthält insbesondere ein (nicht dargestelltes) Schaltnetzwerk der weiter oben erwähnten Art.

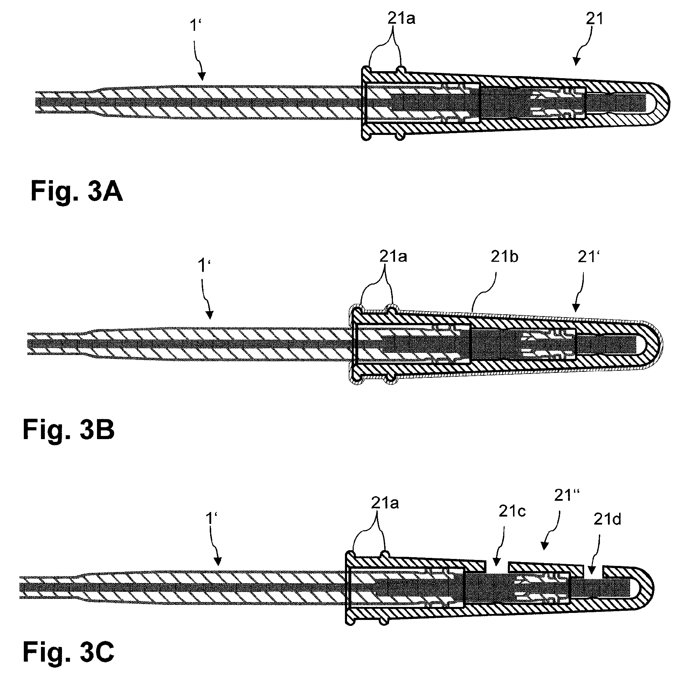

Fig. 3A bis 3C zeigen miteinander verwandte Ausführungen eines hier auch als proximale Elektrodenterminierung bezeichneten Feldentkopplungs-Elementes 21 bzw. 21' bzw. 21", aufgesetzt auf die bereits in Fig. 2 dargestellte Stimulationselektrodenleitung 1' (deren Aufbau in Fig. 3A bis 3C nicht genauer bezeichnet ist). Das Feldentkopplungs-Element ist hier im Wesentlichen eine längliche Kunststoffkappe, die annähernd eine Kegelstumpfform hat und an ihrem distalen Ende Dichtungsringe 21a aufweist. Die Kappe hat einen im Durchmesser in Anpassung an die Elektrodenanschlusskontakte der Leitung 1' abgestuften Innenraum, der einen elektrischen Kontakt zu beiden Elektrodenanschlusskontakten hat und diese niederohmig miteinander und mit dem umliegenden Körpergewebe verbindet.

Die Ausführung nach Fig. 3B unterscheidet sich nach derjenigen von Fig. 3A durch das zusätzliche Vorsehen einer äußeren Isolierschicht 21b, die eine Einstellung der Impedanz zu umgebendem Körpergewebe unabhängig von der Volumenleitfähigkeit des Kunststoffmaterials der Kappe erlaubt. Das Feldentkopplungs-Element (die Kappe) 21" nach Fig. 3C hat Aussparungen 21c und 21d, die im implantierten Zustand der Anordnung der direkten Zutritt von Körperflüssigkeit zu den Elektrodenanschlusskontakten des Elektrodensteckers ermöglichen. Körperflüssigkeit wird hierdurch Teil des Schaltnetzwerkes, welches die Leitungsanschlüsse im Körpergewebe und über dieses mit einer relativ niedrigen Impedanz auch miteinander verbindet. Durch geeignete konstruktive Ausführung der Kappe 21" ist sichergestellt, dass keine Körperflüssigkeit ins Innenlumen der Elektrodenleitung gelangen kann. In dieser Ausführung muss das Kunststoffmaterial der Kappe nicht notwendiger Weise leitfähig sein.

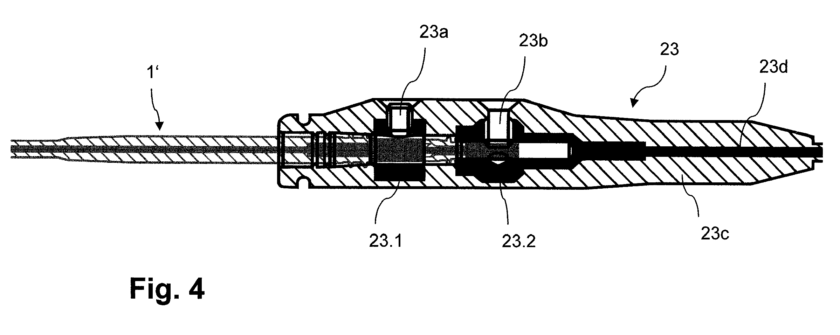

Fig. 4 zeigt, wiederum in Verbindung mit der in Fig. 2 bis 3C gezeigten Stimulationselektrodenleitung 1', als weitere Ausführung des Feldentkopplungs-Elementes ein Terminierungsstück 23 mit einem ersten und zweiten Steckbuchsenkontakt 23.1, 23.2, denen jeweils eine Feststellschraube 23a bzw. 23b zugeordnet ist, in einem Kunststoffkörper 23c. Der Kunststoffkörper 23c enthält weiter ein in Längsrichtung verlaufendes Anschlusskabel 23d, welches zu einem (nicht dargestellten) Schaltnetzwerk des Feldentkopplungs-Elementes führt.

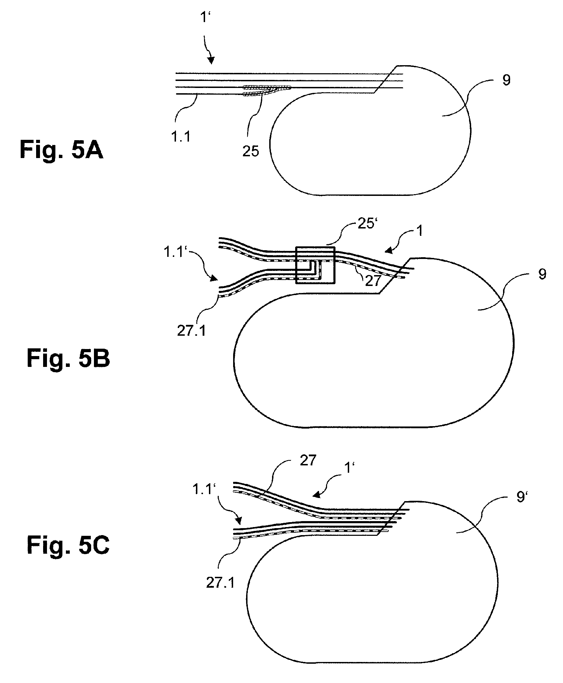

Wie in Fig. 5 skizzenartig dargestellt, kann das Feldentkopplungs-Element auch als Zwischenstück im Verlauf einer (hier mehradrigen) Elektrodenleitung 1 eingesetzt sein. Das Zwischenstück 25 stellt bei dieser Ausführung zugleich ein Verzweigungselement zur Verzweigung in eine zusätzliche (z. B. unbenutzte) Elektrodenleitung 1.1 dar. Die Anschlüsse bzw. Kontakte des Zwischenstücks 25 können Standardanschlüsse (IS1, IS4, HV1) oder spezielle Anschlüsse (hier bezeichnet als IS1+, IS4+, HV1+) sein, die zusätzlich den Anschluss einer weiteren (ggf. ebenfalls dem Ziel einer Störfeldentkopplung dienenden) Leitungsstruktur im Innenlumen der Elektrodenleitung 1 ermöglicht

Fig. 5B zeigt eine Abwandlung der Ausführung nach Fig. 5A, bei der zwei bipolare Elektrodenleitungen 1 bzw. 1.1' über ein modifiziertes Zwischenstück 25' miteinander verschaltet sind. Bei dieser Ausführung hat jede der beiden Elektrodenleitungen einen zusätzlichen nicht medizinisch wirkenden, langgestreckten Feldentkopplungsleiter 27 bzw. 27.1 integriert, und das Zwischenstück 25' verbindet auch diese beiden Feldentkopplungsleiter.

Fig. 5C zeigt eine Ausführung, bei der ein so genanntes aktives Implantat (implantiertes medizinelektronisches Gerät) 9' die Rolle des Feldentkopplungs-Elementes bzw. der proximalen Terminierung der Leitungen 1' und 1.1' übernimmt. Auch hier haben beide Leitungen einen zusätzlichen langgestreckten Feldentkopplungsleiter 27 bzw. 27.1 integriert. Auch nicht benutzte Elektrodenleitungen können mit der Elektronik des Gerätes 9' verbunden sein, so dass das (nicht dargestellte) Schaltnetzwerk zur Feldentkopplung aktiv von dieser Elektronik beeinflusst werden kann. Auf diese Weise kann etwa über einen Steuereingriff über Telemetrie das Schaltnetzwerk in einer bestimmten Weise gesteuert werden, wenn vorauszusehen ist, dass der Patient in den Einwirkungsbereich eines starken externen Feldes gelangen wird, etwa vor einer MR-Untersuchung.

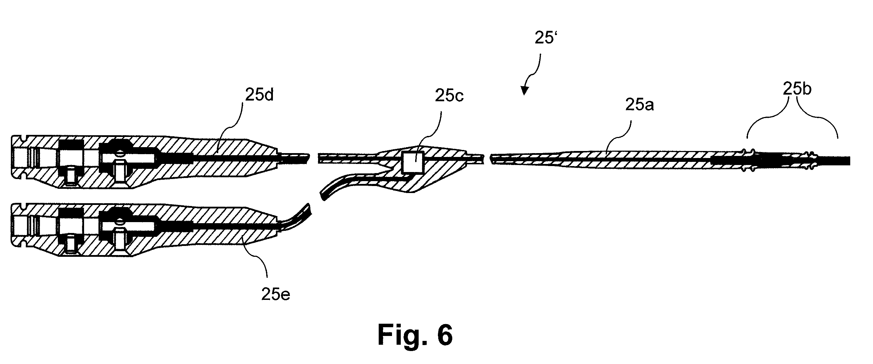

Fig. 6 zeigt eine mechanische Ausführung einer derartigen Verzweigung 25' gemäß Fig. 5B als verzweigter Kunststoff-Leitungskörper 25a, der an einem Ende (proximal) einen zweipoligen Steckerabschnitt 25b, im Verzweigungsbereich ein Schaltnetzwerk 25c und am gegenüberliegenden Ende zwei im Wesentlichen gleichartig ausgeführte, angeformte Steckbuchsenabschnitte 25d, 25e umfasst.

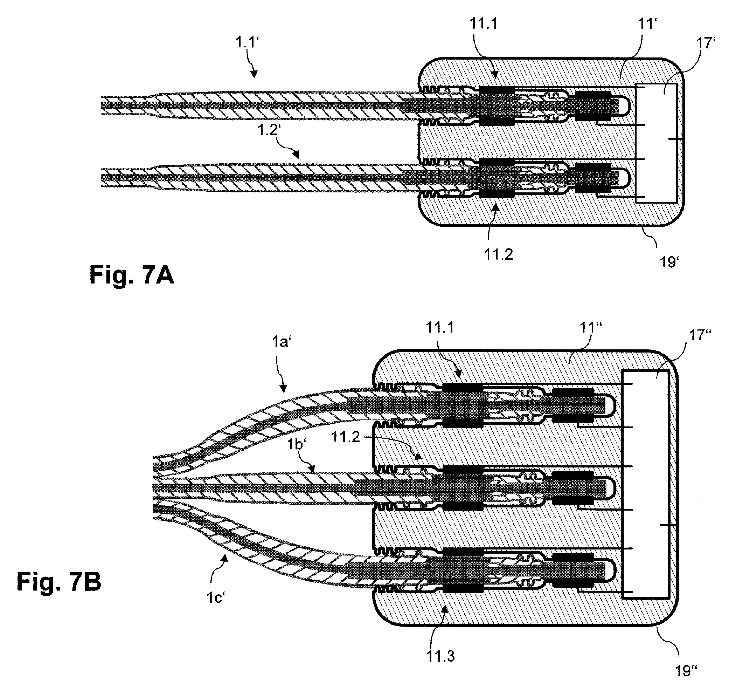

Fig. 7A und 7B zeigen Abwandlungen der Ausführungen nach Fig. 2, bei denen in einem modifizierten Steckbuchsenteil 11' bzw. 11" zwei bzw. drei Buchsenabschnitte 11.1, 11.2 bzw. 11.1 bis 11.3 zum Anschluss zweier Elektrodenleitungen 1.1' und 1.2' bzw. dreier Zweige 1a', 1b' und 1c' vorgesehen sind, deren Kontaktabschnitte jeweils durch ein Schaltnetzwerk 17' bzw. 17" miteinander und mit einem Gehäuse 19' bzw. 19" verbunden sind. Die Steckbuchsenabschnitte der Ausführung nach Fig. 7B müssen nicht in einer Ebene liegen, sondern können eine räumliche Konfiguration haben, und sie können auch konstruktiv unterschiedlich ausgeführt sein.

Die Terminierung ist nicht auf das Vorhandensein von genormten Elektrodenstecken angewiesen. Es sind auch Terminierungen denkbar, die auf Elektrodenzuleitungen gesteckt werden. So könnten z. B. unbenutzte Elektroden gekürzt und anschließend mit einer Terminierung versehen werden. Sie stellen dann Kontakt zu den verschiedenen Leitungen (Wendel, Seil, MRIwire ...) der gekürzten Elektrode her und verbinden diese mit einem elektrischen Netzwerk und ggf. noch dem Körper.

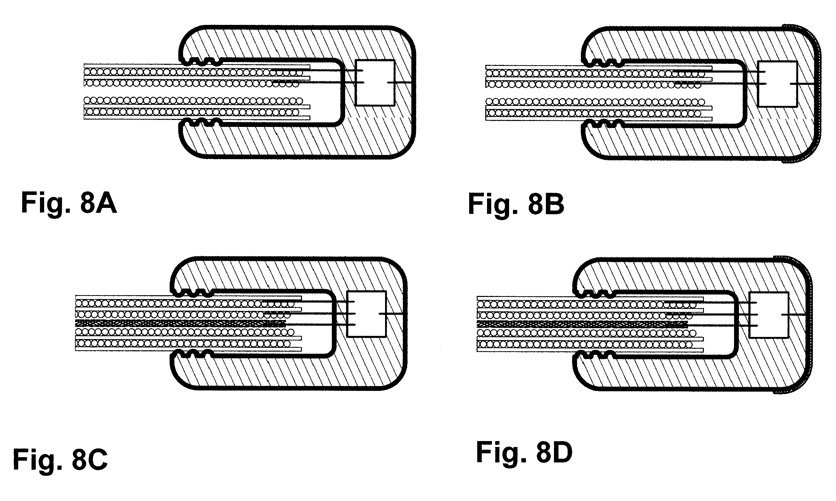

Fig. 8A bis 8D zeigen verschiedene mögliche Ausführungen für bipolare, gewendelte Elektrodenzuleitungen 81. Die Kontaktierung der Elektrodenleiter 81 erfolgt durch leitfähige Strukturen 82, die proximal in die Elektrode gesteckt werden.

Die Varianten nach Fig. 8C und 8D besitzen zusätzlich im Innenlumen einen Feldentkopplungsleiter 83, der ebenfalls von der Terminierung 82' kontaktiert wird. Die Versionen nach Fig. 8B und 8D unterscheiden sich von denen in Fig. 8A und 8C dadurch, dass die Gehäuseoberfläche nicht überall, sondern nur an manchen Stellen 85 leitfähig ist (an der aufgebrachten Schicht).

Die elektrische Verbindung zum Körper muss nicht galvanisch erfolgen, sondern kann kapazitiv durch eine dünne Isolationsschicht hindurch erfolgen. Dies gilt allgemein für die Terminierungen. Der Kontakt zu den Leitern kann auch durch schraubenförmige Strukturen erfolgen, die beispielsweise in die gewendelten Zuleitungen geschraubt werden.

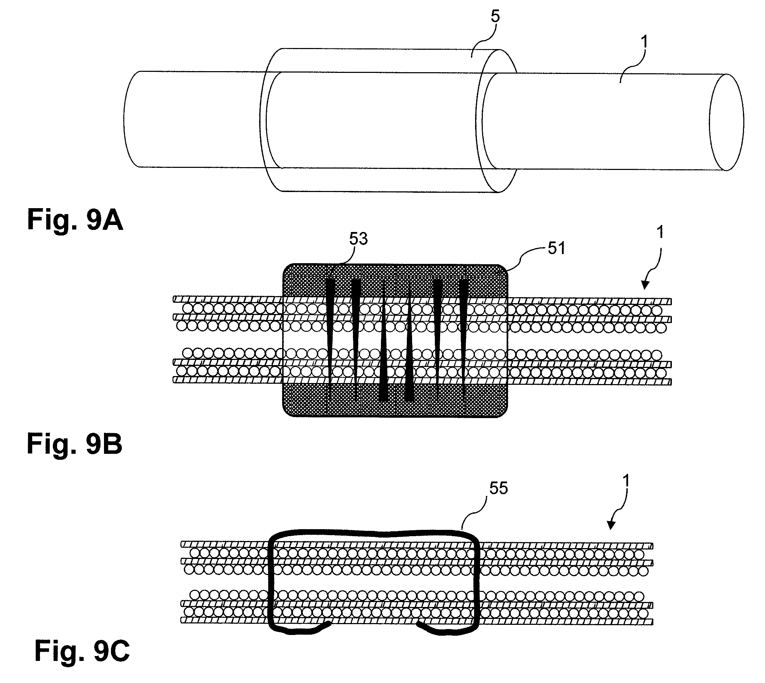

Die Terminierung muss nicht zwingend am Ende einer Elektrode montiert werden. Sie kann, wie in Fig. 9A symbolisch dargestellt, auch entlang einer Elektrode montiert werden (ähnlich einem Ferrit bei einer gewöhnlichen elektrischen Signalleitung):
Das Schnittbild einer solchen Terminierung bei einer bipolaren, gewendelten Elektrodenzuleitung ist in Fig. 9B gezeigt: Ein Grundkörper 51 umgreift die Elektrode 1. Er beinhaltet mehrere leitfähige Strukturen 53, die die beiden Zuleitungen innerhalb der Elektrode miteinander verbinden. Zusammen mit dem leitfähigen Grundkörper wird eine niederohmige Verbindung zwischen den Elektrodenzuleitungen und dem Körper eines Patienten hergestellt. Alternativ kann, wie in Fig. 9C dargestellt, die Vermittlung eines Kontakts zwischen dem Körpergewebe und beiden Elektrodenzuleitungen mit einer länglichen leitfähigen Struktur erfolgen. Die Figur zeigt einen Draht, der zur Klammer 55 geformt ist und so beide Elektrodenzuleitungen mit dem Körpergewebe verbindet. Der Draht sollte aus biokompatiblem Material bestehen. Die leitfähigen Strukturen zum Kontaktieren können auch so ausgeführt sein, dass sie gezielt eine der beiden Elektrodenzuleitungen kontaktieren und diese über ein Netzwerk miteinander und ggf. mit dem Körper verbinden. Der Grundkörper muss nicht leitfähig sein.

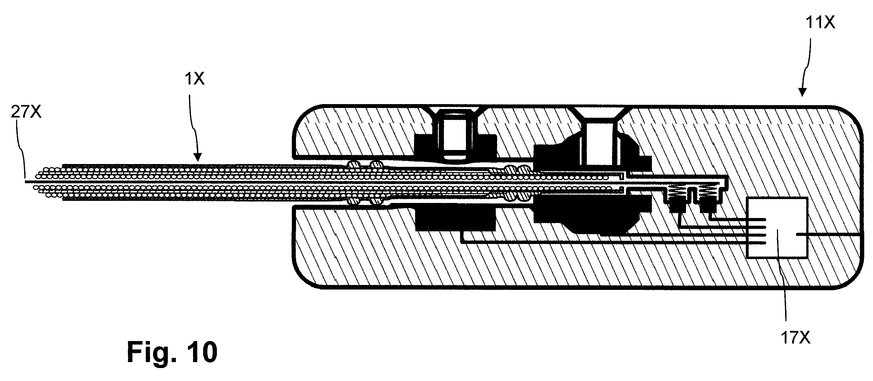

Der Anschluss von zusätzlichen Feldentkopplungsleitern in Elektrodenleitungen an eine Terminierung kann auf verschiedene Arten geschehen. Fig. 10 zeigt eine Terminierung 11X mit einem IS1+-Anschluss, die zusätzlich einen zweiadrigen Feldentkopplungsleiter 27X kontaktiert und alle Einzel-Zuleitungen der Leitung 1X über ein Netzwerk 17X miteinander verbindet. Die Leitung 1X ist als gewendelte, bipolare Leitung dargestellt.

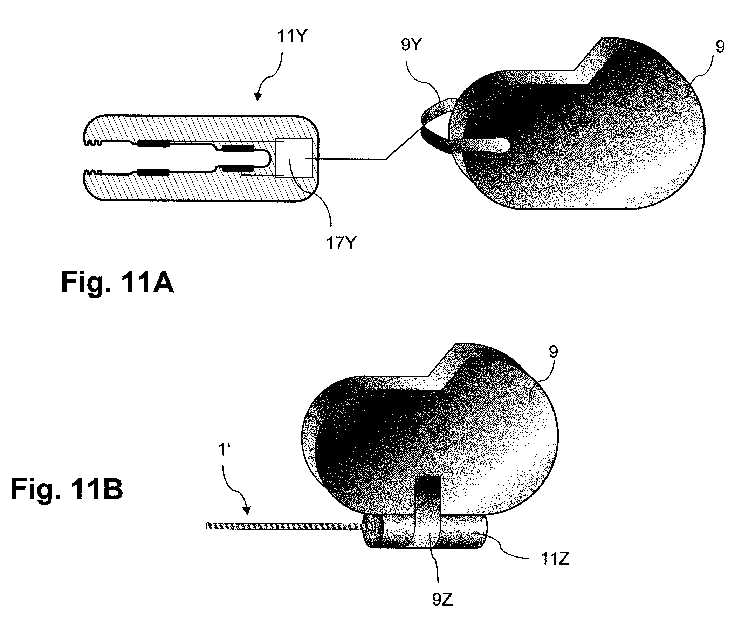

Es ist möglich, die Terminierung nicht nur über einen Elektrodenanschluss mit einem anderen Implantat zu verbinden. So kann z. B. eine Terminierung über eine leitfähige Struktur mit dem Gehäuse eines anderen Implantates verbunden werden. Fig. 11A zeigt beispielhaft einen solchen Aufbau: Eine Terminierung 11Y beinhaltet ein elektrisches Netzwerk 17Y, dessen einer Eingang elektrisch mit dem Gehäuse eines anderen Implantates 9 verbunden ist. Der Gehäusekontakt wird über eine Klammer 9Y gewährleistet.

Bei einer solchen Konstruktion muss die Terminierung selbst keine leitende Oberfläche besitzen, die mit dem Körpergewebe galvanisch oder kapazitiv verbunden ist. Der Kontakt zum Körpergewebe wird über das Gehäuse des zweiten Implantates gewährleistet.

Neben der elektrischen Verbindung einer Terminierung mit einem anderen Implantat ist auch die mechanische Verbindung sinnvoll. So kann die Lage beider Implantate zueinander festgelegt werden. Möglich ist eine direkte Verbindung beider Implantate, oder eine Verbindung, die einen bestimmten Abstand zwischen beiden vorgibt, oder eine biegeschlaffe Verbindung (z. B. mit einem Seil), die für einen nicht zu überschreitenden Maximalabstand beider Implantate sorgt. Die Einschränkung der Freiheitsgrade beider Implantate kann gerade bei der Defibrillation (intern oder extern) von Vorteil sein. Fig. 11B zeigt eine zylindrische Terminierung mit einer Elektrode, die über ein Befestigungselement 9Z direkt mit einem anderen Implantat 9 verbunden ist.

Die Ausführung der Erfindung ist nicht auf die oben beschriebenen Beispiele und hervorgehobenen Aspekte der Erfindung beschränkt, sondern ebenso in einer Vielzahl von Abwandlungen möglich, die im Rahmen fachgemäßen Handelns liegen.

## Patentansprüche

1. Proximale Elektrodenterminierung (21, 21', 21 ", 23) zur Verwendung mit einer implantierbaren Leitung (1') mit einem langgestreckten Leitungskörper und einem sich in Längsrichtung des Leitungskörpers erstreckenden Funktionsleiter (4), der zur Realisierung einer medizinischen Funktion der Leitung wirkt, wobei die proximale Elektrodenterminierung im mit der Leitung verbundenen Gebrauchszustand in elektrischem Kontakt mit dem Funktionsleiter (4) steht und eine Kopplung des Funktionsleiters (4) mit einem äußeren Feld verringert, und wobei die proximale Elektrodenterminierung mindestens zwei Kontakte zur Kontaktierung des Funktionsleiters (4) der Leitung (1') sowie eines weiteren leitenden Elementes im Körper eines Patienten und ein beide Kontakte verbindendes Schaltnetzwerk umfasst, **dadurch gekennzeichnet, dass** die proximale Elektrodenterminierung passend auf einen der Standard-Elektrodenanschlüsse IS1, IS4 oder DF1 ausgebildet ist.

2. Proximale Elektrodenterminierung (21, 21', 21", 23) nach Anspruch 1, wobei das Schaltnetzwerk mindestens ein diskretes passives Bauelement und/oder lineares Bauelement und/oder Halbleiterbauelement und/oder Element aus einem dielektrischem Material und/oder einen Sensor und/oder ein Bauelement aufweist, welches einen Riesenmagnetwiderstand, einen anisotropen Magnetwiderstand, einen kolossalen Magnetwiderstandseffekt oder einen magnetischen Tunnelwiderstand besitzt.

3. Proximale Elektrodenterminierung (21, 21', 21", 23) nach einem der vorangehenden Ansprüche, mit einem elastischen leitfähigen Abschnitt oder Teil, welches im Gebrauchszustand den Funktionsleiter einer angeschlossenen Leitung auch bei mechanischer Krafteinwirkung in Dauerkontakt mit einem weiteren leitenden Element im Körper eines Patienten hält.

4. Proximale Elektrodenterminierung (21, 21', 21", 23) nach einem der vorangehenden Ansprüche 1 bis 3, welches einen Abschnitt oder ein Teil aus einem elektrisch leitfähigen Kunststoff aufweist, welcher/welches insbesondere den ersten mit dem zweiten Kontakt verbindet.

5. Proximale Elektrodenterminierung (21, 21', 21", 23) nach einem der vorangehenden Ansprüche 1 bis 4, welches einen metallischen Abschnitt oder ein metallisches Teil aufweist, wobei die proximale Elektrodenterminierung den ersten mit dem zweiten Kontakt verbindet und in den/das insbesondere ein verstellbares oder elastisches Kontaktierungselement eingefügt ist.

6. Proximale Elektrodenterminierung (21, 21', 21 ", 23) nach Anspruch 5, **dadurch gekennzeichnet, dass** das verstellbare Kontaktierungselement eine Schraubverbindung, und das elastische Kontaktierungselement Lamellen oder Federn umfasst.

7. Proximale Elektrodenterminierung (21, 21', 21", 23) nach einem der vorangehenden Ansprüche, wobei ein aus hoch leitfähigem Material bestehender Oberflächenabschnitt mit einer Isolierschicht bedeckt ist, welche insbesondere aus einem solchen Material besteht und/oder so bemessen ist, dass sie eine vorbestimmte niedrige Impedanz aufweist.

8. Proximale Elektrodenterminierung (21, 21', 21", 23) nach einem der Ansprüche 1 bis 7, welches einen mindestens den ersten mit dem zweiten Kontakt verbindenden Hohlraum zur Aufnahme einer leitfähigen Flüssigkeit aufweist, welcher insbesondere derart ausgebildet ist, dass im Gebrauchszustand Körperflüssigkeit eines Patienten eindringen kann und eine elektrische Verbindung zwischen den Kontakten herstellt.

9. Proximale Elektrodenterminierung (21, 21', 21", 23) nach einem der vorangehenden Ansprüche, ausgebildet zur Verbindung mit mehreren Leitungen und/oder mehreren implantierbaren medizinischen Geräten.

## Claims

1. A proximal electrode termination (21, 21', 21 ", 23) for use with an implantable lead (1') having an elongate lead body and a functional conductor (4), which extends in the longitudinal direction of the lead body and implements a medical function of the lead, wherein the proximal electrode termination is in electrical contact with the functional conductor (4) in the use state connected to the lead and reduces a coupling of the functional conductor (4) to an external field, and wherein the proximal electrode termination comprises at least two contacts for contacting the functional conductor (4) of the lead (1') and also comprises a further conductive element in the body of a patient and a switching network connecting both contacts, **characterised in that** the proximal electrode termination fits on one of the standard electrode connectors IS 1, IS4 or DF1.

2. The proximal electrode termination (21, 21', 21 ", 23) according to claim 1, wherein the switching network has at least one discrete passive component and/or linear component and/or semiconductor component and/or element made of a dielectric material and/or a sensor and/or a component which has a giant magnetoresistance, an anisotropic magnetoresistance, a colossal magnetoresistance effect, or a magnetic tunnel resistance.

3. The proximal electrode termination (21, 21', 21", 23) according to any one of the preceding claims, having a resilient conductive portion or part which, in the use state, holds the functional conductor of a connected lead permanently in contact with a further conductive element in the body of a patient, even under the effect of a mechanical force.

4. The proximal electrode termination (21, 21', 21", 23) according to any one of the preceding claims 1 to 3, which has a portion or a part made of an electrically conductive plastic which in particular connects the first contact to the second contact.

5. The proximal electrode termination (21, 21', 21", 23) according to any one of the preceding claims 1 to 4, which has a metal portion or a metal part, wherein the proximal electrode termination connects the first contact to the second contact, and into which metal portion or metal part in particular an adjustable or resilient contacting element is inserted.

6. The proximal electrode termination (21, 21', 21", 23) according to claim 5, **characterised in that** the adjustable contacting element comprises a screw connection and the resilient contacting element comprises lamellas or springs.

7. The proximal electrode termination (21, 21', 21", 23) according to any one of the preceding claims, wherein a surface portion consisting of highly conductive material is covered by an insulating layer which in particular consists of such a material and/or is dimensioned such that it has a predetermined low impedance.

8. The proximal electrode termination (21, 21', 21", 23) according to any one of the preceding claims 1 to 7, which has a cavity, connecting at least the first contact to the second contact, for receiving a conductive fluid, which cavity in particular is designed such that, in the use state, bodily fluid of a patient can infiltrate and produces an electrical connection between the contacts.

9. The proximal electrode termination (21, 21', 21", 23) according to any one of the preceding claims, designed for connection to a plurality of leads and/or a plurality of implantable medical devices.

## Revendications

1. Terminaison d'électrode proximale (21, 21', 21", 23) pour l'utilisation avec une ligne implantable (1') avec un corps de ligne étiré en longueur et un conducteur fonctionnel (4) s'étirant dans la direction longitudinale du corps de ligne, qui agit pour la réalisation d'une fonction médicinale de la ligne, où la terminaison d'électrode proximale, dans un état d'utilisation relié avec la ligne, est en contact électrique avec le conducteur fonctionnel (4) et resserre un couplage du conducteur fonctionnel (4) avec un champ extérieur, et où la terminaison d'électrode proximale comprend au moins deux contacts pour la mise en contact du conducteur fonctionnel (4) de la ligne (1') ainsi qu'un autre élément conducteur dans le corps d'un patient et un réseau de commutation reliant les deux contacts, **caractérisée en ce que** la terminaison d'électrode proximale est conçue pour s'ajuster à l'un des raccords d'électrodes standard IS1, IS4 ou DF1.

2. Terminaison d'électrode proximale (21, 21',21",23) selon la revendication 1, où le réseau de commutation présente au moins un composant passif discret, et/ou un composant linéaire, et/ou un composant semi-conducteur, et/ou un élément à base d'un matériau diélectrique, et/ou d'un capteur, et/ou d'un composant, lequel possède une magnétorésistance géante, une magnétorésistance anisotrope, une effet magnéto résistant colossal ou une magnétorésistance à effet tunnel.

3. Terminaison d'électrode proximale (21, 21',21",23) selon l'une des revendications précédentes, avec un segment conducteur élastique ou une partie, laquelle, à l'état d'utilisation, conserve en contact permanent le conducteur fonctionnel d'une ligne raccordée dans une connexion raccordée, également sous l'effet d'une force mécanique avec un autre élément conducteur dans le corps d'un patient.

4. Terminaison d'électrode proximale (21, 21',21",23) selon l'une des revendications précédentes 1 à 3, laquelle présente un segment ou une partie à base d'un plastique conducteur électriquement, lequel / laquelle relie notamment le premier contact avec le second.

5. Terminaison d'électrode proximale (21, 21',21",23) selon l'une des revendications précédentes 1 à 4, laquelle présente un segment métallique ou une partie métallique, où la terminaison d'électrode proximale relie le premier contact avec le second et dans lequel / laquelle est notamment incorporé un élément de contact réglable ou élastique.

6. Terminaison d'électrode proximale (21, 21', 21", 23) selon la revendication 5, **caractérisée en ce que** l'élément de contact réglable est une liaison par vissage, et l'élément de contact élastique comprend des lamelles ou des ressorts.

7. Terminaison d'électrode proximale (21, 21', 21 ", 23) selon l'une des revendications précédentes, où un segment de surface constitué d'un matériau hautement conducteur est recouvert avec une couche d'isolation, laquelle est notamment constituée d'un matériau et/ou est dimensionnée de sorte qu'elle présente une impédance faible prédéfinie.

8. Terminaison d'électrode proximale (21, 21', 21", 23) selon l'une des revendications 1 à 7, laquelle présente au moins un espace creux reliant le premier contact avec le second pour l'admission d'un liquide conducteur, lequel est en particulier conçu pour pouvoir pénétrer dans le liquide corporel d'un patient lors de l'état d'utilisation et établit une liaison électrique entre les contacts.

9. Terminaison d'électrode proximale (21, 21',21",23) selon l'une des revendications précédentes, conçue pour la liaison avec plusieurs lignes et/ou plusieurs appareils médicinaux implantables.
